# FASCICULE DE BREVET EUROPEEN

(11) **EP 2 429 378 B1**
(45) Date de publication et mention de la délivrance du brevet: **23.04.2014**
(21) Numéro de dépôt: 10727775.8
(22) Date de dépôt: 07.05.2010
(51) Int. Cl.: A61B 3/113, G06F 3/01

(54) **PAIRE DE LUNETTES OPHTALMIQUES ADAPTEE POUR CARACTERISER UNE CONVERGENCE DES YEUX D'UN PORTEUR**
OPHTHALMISCHE BRILLE ZUR CHARAKTERISIERUNG EINER KONVERGENZ DER AUGEN EINES TRÄGERS
OPHTHALMIC SPECTACLES FOR CHARACTERIZING A CONVERGENCE OF THE EYES OF A WEARER

(30) Priorité: 12.05.2009 FR 0953126
(43) Date de publication de la demande: 21.03.2012
(73) Titulaire: Essilor International (Compagnie Générale d'Optique), 94220 Charenton-Le-Pont (FR)
(72) Inventeur: BONNIN, Thierry, F-94220 Charenton-Le-Pont (FR); BROSSIER, Thibault, F-94220 Charenton-Le-Pont (FR); ROUSSEAU, Benjamin, F-94220 Charenton-Le-Pont (FR)
(74) Mandataire: Cabinet Plasseraud
(86) Numéro de dépôt international: PCT/FR2010/050893
(87) Numéro de publication internationale: WO 2010/130931

(56) Documents cités:
- EP-A1- 0 733 338
- WO-A1-99/18868
- US-A- 3 689 135
- US-A- 4 300 818
- US-A- 5 070 883
- US-B1- 6 283 954

## Description

La présente invention concerne une paire de lunettes ophtalmiques qui est adaptée pour caractériser une convergence des yeux d'un porteur de cette paire de lunettes. Elle concerne aussi un verre d'une telle paire de lunettes.

De nombreux systèmes existent déjà, qui permettent de détecter les mouvements oculaires d'un sujet en temps réel. Par exemple, le document US 5,966,197 décrit un système de chirurgie oculaire par ablation laser, qui permet de détecter et de compenser les mouvements oculaires d'un patient. Ce système forme une image de l'oeil, et la position de celui-ci en rotation dans l'orbite est repérée en identifiant la position du limbe dans l'image. On rappelle que le limbe est la limite entre la sclérotique et l'iris de l'oeil, et qu'il peut être facilement détecté dans une image de l'oeil en lumière du domaine proche infrarouge, pour des longueurs d'onde qui sont comprises entre 900 nm (nanomètre) et 1200 nm. En effet, la sclérotique possède un coefficient d'intensité de réflexion lumineuse qui est élevé, de l'ordre de 90%, dans ce domaine spectral, alors que l'iris possède un coefficient d'intensité de réflexion plus faible, de l'ordre de 40%.

Le document US 4,300,818 qui est considéré comme représentant l'état de la technique le plus proche décrit une paire de lunettes ophtalmiques, comprenant une monture et deux verres maintenus dans la monture pour être situés respectivement devant les yeux d'un porteur des dites lunettes, et permettant une vision distincte pour le porteur à travers chaque verre et la paire de lunettes comprenant pour chaque verre une source d'un rayonnement sélectionnée pour que ledit rayonnement soit réfléchi par la sclérotique et par l'iris de chaque oeil du porteur conformément à des coefficients d'intensité de réflexion différents respectivement pour la sclérotique et pour l'iris et un détecteur disposé pour mesurer des intensités respectives de parties du rayonnement produit par la source, réfléchies respectivement dans des zones oculaires dans chacune desquelles se déplace une portion droite ou gauche d'une limite entre la sclérotique et l'iris de l'oeil situé derrière ledit verre lors de mouvements oculaires.

D'autres systèmes de détection des mouvements oculaires existent aussi, qui sont destinés à être utilisés par des patients en état conscient. Certains de ces autres systèmes sont constitués d'appareils à placer sur la tête du sujet, et forment des images des deux yeux du sujet en même temps que celui-ci regarde des objets de son environnement. Mais bien qu'un sujet qui est équipé d'un de ces appareils conserve sa mobilité, il ne s'agit pas de dispositifs qui soient utilisables dans la vie courante, mais de dispositifs dont l'utilisation est limitée à des séances de mesures pratiquées sur le sujet.

Or certaines applications nécessitent des systèmes de détection des mouvements oculaires, qui soient compatibles avec une utilisation nomade. Il existe donc un besoin pour un système de détection des mouvements oculaires qui soit à la fois léger et esthétique, et qui ne provoque pas de gêne

pour le sujet lors d'une utilisation prolongée dans la vie courante. En outre, un tel système est de préférence peu onéreux, pour permettre d'équiper un grand nombre d'individus. Mais il n'existe pas à ce jour de systèmes de détection des mouvements oculaires qui réponde à ces besoins de façon satisfaisante.

Un but de la présente invention est donc de fournir un système de détection des mouvements oculaires qui soit compatible avec une utilisation nomade dans la vie courante.

Un autre but de l'invention est de fournir une caractérisation en temps réel de l'éloignement d'un objet qui est observé par un utilisateur du système de détection des mouvements oculaires.

Un autre but encore de l'invention est de fournir une caractérisation de la convergence des yeux de l'utilisateur, et de ses mouvements oculaires qui déterminent les variations de cette convergence.

Pour cela, l'invention propose une paire de lunettes ophtalmiques qui comprend une monture et deux verres maintenus dans la monture pour être situés respectivement devant les yeux d'un porteur des lunettes, et qui permet une vision distincte pour le porteur à travers chaque verre, la paire de lunettes comprenant pour chaque verre :
- au moins une source d'un rayonnement pouvant être un rayonnement infrarouge, qui est sélectionnée pour que ce rayonnement soit réfléchi par la sclérotique et par l'iris de chaque oeil du porteur conformément à des coefficients d'intensité de réflexion différents respectivement pour la sclérotique et pour l'iris ;
- au moins un détecteur, qui est disposé pour mesurer des intensités respectives de parties du rayonnement produit par la source, réfléchies respectivement dans des zones oculaires dans chacune desquelles se déplace une portion droite ou gauche d'une limite entre la sclérotique et l'iris de l'oeil situé derrière le verre lors de mouvements oculaires ; et
- au moins quatre sections de sortie ou d'entrée de rayonnement qui sont disposées dans le verre, chaque section de sortie étant disposée pour diriger une partie du rayonnement produit par la source vers au moins une des zones oculaires où se déplacent les portions droite et gauche de la limite entre la sclérotique et l'iris, et chaque section d'entrée étant disposée pour collecter une partie du rayonnement qui est réfléchie dans l'une de ces zones oculaires.

Selon une première caractéristique de l'invention, ces source(s), détecteur(s) et sections de sortie et d'entrée de rayonnement sont en outre disposés de façon à former au moins quatre chemins de rayonnement qui comportent chacun une réflexion sur l'oeil du porteur situé derrière le verre, dans l'une des zones oculaires où se déplacent les portions droite et gauche, respectivement pour l'un et l'autre de ces chemins, de la limite entre la sclérotique et l'iris de l'oeil. La paire de lunettes comprend alors des moyens pour séparer les parties de rayonnement qui sont transmises entre ladite au moins une source de rayonnement et ledit au moins un détecteur respectivement par des chemins de rayonnement différents.

Selon une seconde caractéristique de l'invention, la paire de lunettes comprend aussi une unité de calcul qui est adaptée pour caractériser une convergence des yeux du porteur vers un point d'observation commun, en fonction des intensités qui sont mesurées simultanément pour les deux verres.

Selon une troisième caractéristique de l'invention, pour chaque verre, les dits au moins une source, au moins un détecteur et au moins quatre zones de sortie ou d'entrée de rayonnement sont disposés de façon à former au moins deux paires de chemins distincts pour le rayonnement, avec deux premières sections de sortie de rayonnement qui sont disposées dans chaque verre à une même première hauteur, et deux secondes sections de sortie de rayonnement qui sont disposées dans chaque verre à une même seconde hauteur, différente de la première hauteur. Ils sont disposés en outre pour que les zones oculaires où se déplacent les portions droite et gauche, respectivement pour l'un et l'autre des chemins de chaque paire, de la limite entre la sclérotique et l'iris de l'oeil du porteur situé derrière le verre, soient situées à une même hauteur de zones oculaires qui est associée à cette paire, et pour que les deux paires de chemins soient associées à des hauteurs respectives de zones oculaires qui sont distinctes et communes aux deux verres. L'unité de calcul est alors adaptée pour caractériser la convergence des yeux du porteur en fonction des intensités qui sont mesurées simultanément pour les parties de rayonnement transmises par les chemins associés à une même des hauteurs de zones oculaires, cette hauteur étant sélectionnée par l'unité de calcul pour les deux verres.

Ainsi, la présente invention fournit un système de détection des mouvements oculaires d'un sujet, qui se présente sous forme d'une paire de lunettes, avec des sections de sortie et d'entrée de rayonnement qui sont intégrées dans les verres. Le système est donc léger et produit un encombrement très réduit, grâce à sa présentation sous forme d'une paire de lunettes. Il peut donc être utilisé dans la vie courante, notamment lorsque l'utilisateur se déplace. En particulier, un porteur qui est équipé d'une paire de lunettes selon l'invention conserve une liberté de mouvement totale.

En outre, étant donné que les sections de sortie et d'entrée de rayonnement sont intégrées dans chaque verre, la paire de lunettes est dépourvue de tout réflecteur additionnel et de tout système de prise de vue dirigé vers les yeux du porteur, qui serait situé en avant de son visage en plus des verres de lunettes. Un système de détection de mouvements oculaires selon l'invention est donc esthétique sur le visage du porteur, et ne provoque aucune gêne visuelle pour celui-ci.

En outre, la détection des mouvements oculaires qui est réalisée en utilisant une paire de lunettes selon l'invention est fondée sur la détection des positions de portions droite et gauche du limbe de chaque oeil. Pour cela, au moins quatre faisceaux de rayonnement sont dirigés à partir de chaque verre vers les zones de déplacement des portions droite et gauche du limbe de l'oeil correspondant du porteur, et les intensités respectives de ces faisceaux après réflexion sur chaque oeil dans ces zones sont mesurées. Chaque faisceau possède un chemin de rayonnement différent. Son intensité après réflexion, lorsqu'il traverse en retour l'une des sections d'entrée du verre, varie en fonction de la position en rotation des yeux, puisque chaque faisceau est plus ou moins réfléchi selon qu'il atteint la surface de l'oeil plutôt dans la sclérotique ou plutôt dans l'iris. La convergence des yeux du porteur vers un point qui est regardé par celui-ci à un instant donné est alors déduite d'une comparaison entre les positions des limbes des deux yeux. De cette façon, la convergence des yeux du porteur peut être déterminée quelque soit l'angle de la direction d'un objet qui est regardé par le porteur à partir de son visage dans un plan horizontal. Une caractérisation de la distance d'éloignement d'un objet qui est observé par le porteur de la paire de lunettes peut alors être déduite en temps réel de la convergence de ses yeux qui est déterminée.

Par ailleurs, grâce à la disposition des sections de sortie de rayonnement par paires à l'intérieur de chaque verre, en fonction de la hauteur des zones oculaires de réflexion du rayonnement, l'invention permet de déterminer la convergence des yeux du porteur quelque soit l'angle de la direction de l'objet qui est regardé par rapport au visage du porteur dans un plan vertical, appelé hauteur du regard. Ceux des chemins des deux verres qui sont associés à une même des hauteurs de zones oculaires sont utilisés pour déterminer la convergence des yeux du porteur à un instant donné.

Dans divers modes de réalisation de l'invention, certains des perfectionnements suivants peuvent être utilisés séparément ou en combinaison :
- l'unité de calcul peut être adaptée pour sélectionner une des hauteurs de zones oculaires pour caractériser la convergence des yeux du porteur, en fonction d'une valeur d'un rapport signal/bruit de certaines au moins des intensités qui sont mesurées ;
- la paire de lunettes peut comprendre autant de sections de sortie de rayonnement que de sections d'entrée de rayonnement pour chaque verre, chacune de ces sections de sortie étant juxtaposée dans le verre avec l'une des sections d'entrée pour former avec elle un couple séparé d'émission-réception de rayonnement, et chaque couple d'émission-réception étant disposé devant une des zones oculaires où se déplace l'une des portions droite et gauche de la limite entre la sclérotique et l'iris de l'oeil du porteur qui est situé derrière ce verre lors des mouvements oculaires ;
- pour chaque verre, les sections de sortie de rayonnement peuvent être disposées sous forme de deux colonnes qui sont situées respectivement dans des parties droite et gauche de ce verre, avec chaque section de sortie de la colonne qui est située dans la partie droite du verre appartenant à un chemin de rayonnement qui comporte une réflexion sur l'oeil du porteur dans la zone oculaire où se déplace la portion droite de la limite entre la sclérotique et l'iris de cet oeil, et chaque section de sortie de la colonne qui est située dans la partie gauche du verre appartenant à un chemin de rayonnement qui comporte une réflexion sur l'oeil du porteur dans la zone oculaire où se déplace la portion gauche de la limite entre la sclérotique et l'iris de l'oeil ; et
- la (les) source(s), les sections de sortie, la (les) section(s) d'entrée et le (les) détecteur(s) qui sont associés à l'un au moins des verres peuvent être disposés de sorte qu'au moins deux chemins différents de rayonnement traversant des sections de sortie différentes pénètrent par une même section d'entrée commune à ces chemins, et les moyens pour séparer les parties de rayonnement en fonction des chemins de rayonnement peuvent être adaptés pour synchroniser des mesures réalisées par le (les) détecteur(s) conformément à des modulations différentes qui sont appliquées aux rayonnements transmis par les chemins différents.

Eventuellement, une paire de lunettes selon l'invention peut comprendre en outre des moyens pour varier une caractéristique de l'un au moins des verres, en fonction d'un résultat de la convergence des yeux du porteur qui est caractérisée par l'unité de calcul. En particulier, les moyens pour varier la caractéristique de l'un des verres peuvent être compris dans ce verre. Par exemple, chaque verre peut être du type unifocal avec une puissance optique qui est ajustable en fonction de la distance d'observation, cette dernière étant déduite de la convergence des deux yeux du porteur. Un porteur presbyte peut ainsi avoir une correction visuelle qui est adaptée pour chaque distance d'observation dans toute la surface des verres, et qui est dépourvue d'astigmatisme résiduel.

Une paire de lunettes selon l'invention peut aussi être utilisée avantageusement en orthoptie, lors de séances de rééducation visuelle. En effet, la paire de lunettes permet de soumettre le patient, qui est équipé de celle-ci, à des exercices de rééducation plus adaptés et plus contrôlés.

L'invention propose aussi un verre de lunettes qui est adapté pour être assemblé dans une paire de lunettes telle que décrite précédemment. Un tel verre comprend :
- au moins une source d'un rayonnement pouvant être un rayonnement infrarouge, chaque source étant intégrée au verre et sélectionnée pour que le rayonnement soit réfléchi par la sclérotique et par l'iris d'un oeil d'un porteur du verre, conformément à des coefficients d'intensité de réflexion différents respectivement pour la sclérotique et pour l'iris ;
- au moins un détecteur de ce rayonnement, chaque détecteur étant intégré au verre et disposé pour mesurer une intensité d'une partie du rayonnement produit par l'une des sources, qui est réfléchie par l'oeil dans une zone oculaire dans laquelle se déplace une portion droite ou gauche d'une limite entre la sclérotique et l'iris de l'oeil lors de mouvements oculaires ;
- des pistes conductrices transparentes, qui sont intégrées au verre et qui relient électriquement des bornes de chaque détecteur et de chaque source, et qui sont dirigées radialement dans une partie périphérique du verre ; et
- au moins quatre sections de sortie ou d'entrée de rayonnement qui sont disposées dans le verre pour former des chemins de rayonnement avec l'oeil d'un porteur de ce verre de lunettes comme décrit précédemment.

La disposition radiale des pistes dans la partie périphérique du verre permet notamment de prévoir des contacts électriques dans la monture à des emplacements déterminés, pour relier électriquement les pistes du verre à d'autres composants électroniques portés par la monture. En outre, cette disposition radiale permet que le verre soit détouré selon la forme de son logement dans la monture, quelque soit cette forme, en conservant la possibilité de prévoir les emplacements des contacts électriques de la monture.

D'autres particularités et avantages de la présente invention apparaîtront dans la description ci-après d'exemples de réalisation non limitatifs, en référence aux dessins annexés, dans lesquels :
- les figures 1 a et 1b sont des vues en perspective et en plan, respectivement, montrant l'utilisation d'une paire de lunettes selon un premier mode de réalisation de l'invention ;
- les figures 2a et 2b sont des vues agrandies de face et en plan, respectivement, illustrant le premier mode de réalisation de l'invention ;
- les figures 2c et 2d correspondent à la figure 2b, respectivement pour deux positions différentes d'un oeil d'un porteur de la paire de lunettes ;
- la figure 3 correspond à la figure 2a pour un deuxième mode de réalisation de l'invention ;
- les figures 4a et 4b sont des vues respectives en plan et en section transversale d'un verre selon l'invention ;
- les figures 5 et 6 illustrent deux types différents de modes de réalisation de l'invention ;
- les figures 7a à 7d correspondent respectivement aux figures 2a à 2d pour un troisième mode de réalisation de l'invention ; et
- la figure 8 correspond à la figure 3 pour un quatrième mode de réalisation de l'invention.

Pour raison de clarté, les dimensions des éléments qui sont représentés dans ces figures ne sont pas en proportion avec des dimensions réelles, ni avec des rapports de dimensions réels. En outre, des références identiques sur des figures différentes désignent des éléments identiques ou qui ont des fonctions identiques.

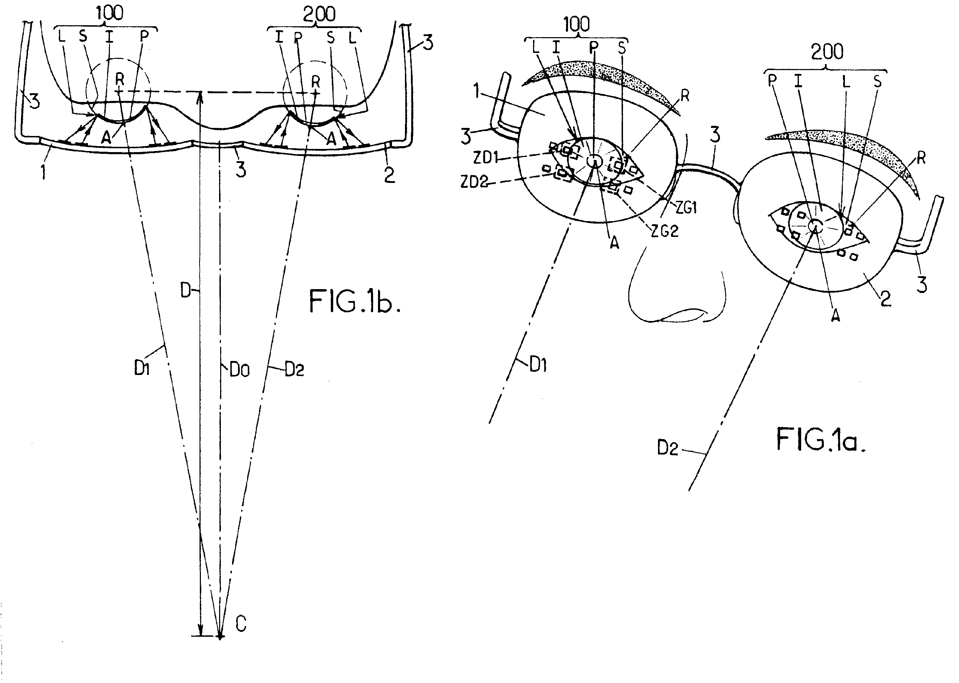

Conformément aux figures 1 a et 1 b, une paires de lunettes comprend une monture 3 et deux verres ophtalmiques, qui sont référencés 1 et 2 pour le verre droit et le verre gauche, respectivement. La monture 3 maintient les verres 1 et 2 dans des positions relatives qui sont fixes, et permet de les placer devant les yeux d'un porteur de la paire de lunettes d'une façon qui est constante lors de durées de port successives. Les verres 1 et 2 peuvent être assemblés définitivement dans la monture 3, en utilisant l'un des procédés d'assemblage connus des opticiens. Alternativement, les verres 1 et 2 peuvent être ajoutés par l'invention à une paire de lunettes initiale qui comprend la monture 3. La paire de lunettes initiale peut être une paire de lunettes de protection solaire et/ou de correction ophtalmique. Dans ce cas, les verres 1 et 2 peuvent être maintenus sur la paire de lunettes initiale de façon amovible, par exemple par clipsage.

Les références 100 et 200 désignent les yeux du porteur, respectivement son oeil droit et son oeil gauche. Pour chaque oeil 100, 200 du porteur, les références S, I, P, L et R désignent la sclérotique, l'iris, la pupille, le limbe et le centre de rotation de l'oeil. De façon connue, l'iris I est une couronne circulaire dont le diamètre interne est variable et détermine la taille de la pupille P, et dont le diamètre externe est constant. Le limbe L est la limite externe de l'iris I, entre cet iris et la sclérotique S. C'est donc un cercle de taille constante qui est fixe par rapport à l'oeil correspondant lorsque ce dernier tourne autour de son centre de rotation R. Visuellement, le limbe L est la limite circulaire entre la sclérotique S qui est blanche et l'iris coloré I.

Pour chaque oeil 100, respectivement 200, l'axe D1, resp. D2, qui passe par le centre de rotation R et le centre A de la pupille P correspondante est l'axe optique de cet oeil. Le centre A de la pupille P est aussi le sommet du cristallin, et est appelé «apex». L'axe optique D1, resp. D2, est fixe par rapport à l'oeil 100, resp. 200, de sorte qu'il est entraîné en rotation avec le limbe L. Les axes optiques D1 et D2 des yeux 100 et 200 convergent vers un point commun C (figure 1 b), qui est appelé point de convergence des yeux et qui est l'emplacement d'un objet regardé par le porteur à un instant donné. La direction moyenne D0 des axes optiques D1 et D2 est la direction de regard du porteur à cet instant. Usuellement, la direction de regard D0 relie un milieu du segment entre les deux centres de rotation oculaire R et le point de convergence C. La distance d'observation, qui est notée D sur la figure 1b, est l'éloignement du point de convergence C par rapport aux centres de rotation R.

La présente invention, qui est décrite maintenant dans des modes de réalisation particuliers, permet de déterminer la position du point de convergence C par rapport au visage du porteur. Dans ces modes de réalisation, cette position du point C est déterminée à partir d'une détection de la position en rotation de chaque oeil 100, 200 par rapport au verre correspondant 1, 2. Ainsi, chaque verre 1, 2 selon l'invention permet de déterminer la position angulaire de l'axe optique D1, D2 de l'oeil 100, 200 correspondant. Le point de convergence C, avec la distance d'observation D si nécessaire, est alors déduit des positions respectives des deux axes optiques D1 et D2.

Pour repérer l'axe optique de chaque oeil, on utilise deux angles α et β, appelés respectivement hauteur et excentricité. La hauteur α est usuellement identique pour les deux yeux 100 et 200, et est l'angle entre chaque axe optique D1 ou D2 et un plan de référence qui est horizontal lorsque la tête du porteur est elle-même verticale. L'excentricité β de l'axe optique D1 ou D2 de chaque oeil est l'angle entre cet axe et un plan médian du visage, qui est vertical lorsque la tête du porteur est elle-même verticale. L'excentricité β possède en général des valeurs qui sont distinctes pour les deux yeux à un même instant, et l'écart entre ces deux valeurs détermine la convergence des yeux, c'est-à-dire la distance d'observation D.

Pratiquement, l'excentricité de l'axe optique D1, D2 de chaque oeil 100, 200 est déterminée à partir de la position du limbe L de cet oeil. Plus précisément, des positions respectives de bords droit et gauche du limbe L sont déterminées, en mesurant des intensités de rayonnements qui sont réfléchis par l'oeil dans des zones latérales droite et gauche de celui-ci. Pour cela, chaque verre est pourvu de sections d'émission du rayonnement, appelées sections de sortie, qui permettent de diriger un ou plusieurs faisceaux de rayonnement vers les zones latérales de l'oeil dans lesquelles se déplacent les portions droite et gauche du limbe lorsque l'oeil tourne. Il est aussi pourvu de sections de collecte, appelées sections d'entrée, pour collecter des parties de ce rayonnement qui ont été réfléchies dans les zones latérales de l'oeil. Les sections de sortie sont alimentées en rayonnement par au moins une source de rayonnement, et les sections d'entrée sont reliées optiquement à au moins un détecteur pour mesurer les intensités des parties du rayonnement qui sont collectées respectivement par ces sections d'entrée.

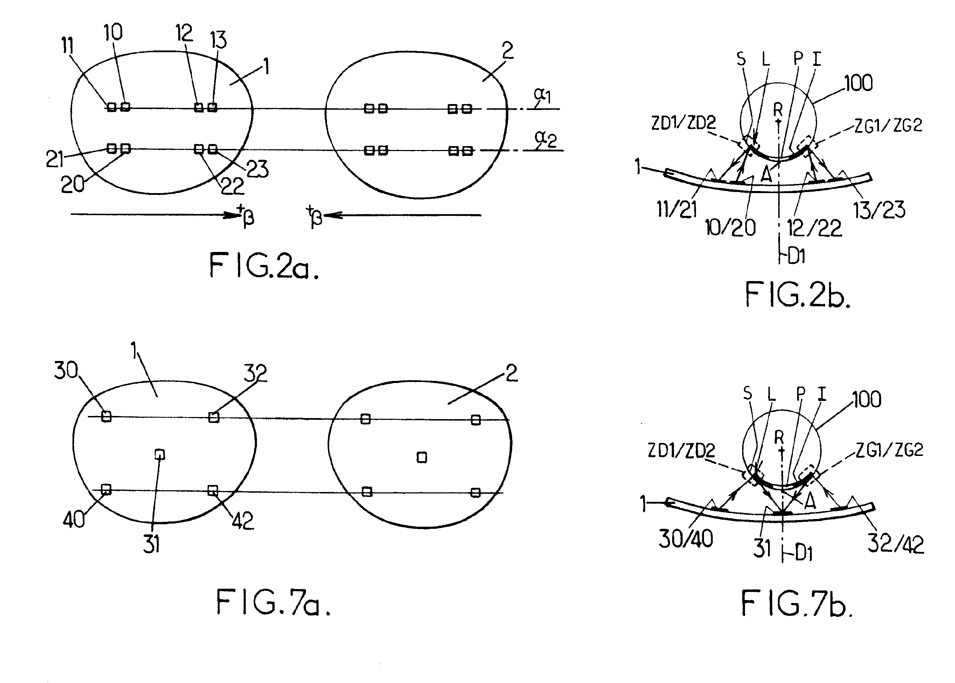

Sur le verre droit 1 des figures 2a à 2d, un premier couple d'émission-réception est constitué par la section de sortie 10 et par la section d'entrée 11. Les sections 10 et 11 sont juxtaposées, par exemple selon une direction horizontale, avec un décalage qui peut être compris entre 0,1 et 3 mm (millimètre). Un deuxième couple d'émission-réception est constitué par une autre section de sortie 12 et par une autre section d'entrée 13. Les couples 10/11 et 12/13 peuvent avoir des constitutions qui sont identiques. Ils sont situés dans le verre 1 à des valeurs de la hauteur α qui sont égales, notées α₁. Ils sont situés en outre à des valeurs d'excentricité β qui sont différentes : le couple d'émission-réception 10/11 est situé en vis-à-vis de la zone latérale droite ZD1 de l'oeil où se déplace la portion droite du limbe L, et le couple d'émission-réception 12/13 est situé en vis-à-vis de la zone latérale gauche ZG1 de l'oeil où se déplace la portion gauche du limbe L. Chaque couple d'émission-réception détermine donc un chemin de rayonnement qui est indiqué sur les figures 2b à 2d, à partir de la section de sortie 10 ou 12 correspondante et jusqu'à la section d'entrée 11 ou 13 correspondante, et qui comprend un point de réflexion du rayonnement dans la zone ZD1 ou ZG1 correspondante. Ainsi, dans un tel mode de réalisation de l'invention, les différents chemins de rayonnement qui sont utilisés pour déterminer la position de l'oeil en rotation sont séparés par la façon de disposer les sections de sortie et d'entrée du rayonnement dans le verre.

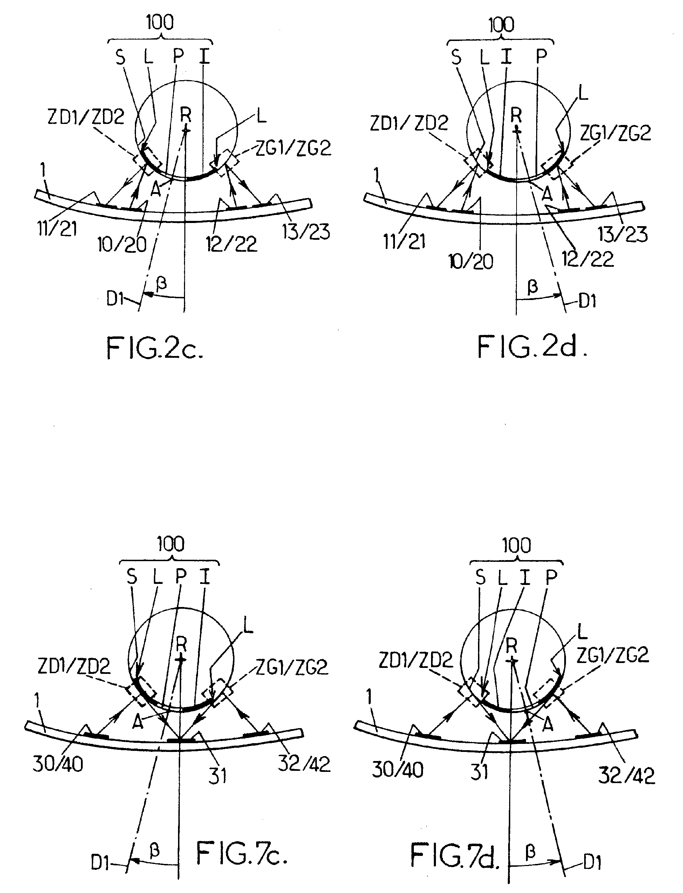

Les figures 2b, 2c et 2d représentent respectivement des positions en rotation de l'oeil droit 100, pour lesquelles l'axe optique D1 correspond à une valeur de l'excentricité β qui est nulle (figure 2b), négative ou orientée vers le côté temporal (figure 2c) et positive ou orientée vers le côté nasal (figure 2d). Lorsque le rayonnement utilisé possède une longueur d'onde qui est comprise entre 900 et 1200 nm, la différence entre l'intensité de réflexion de l'iris, qui est faible, et celle de la sclérotique, qui est plus élevée, permet de détecter la position de l'oeil 100. Ainsi, les réflexions qui sont détectées respectivement dans les zones latérales ZD1 et ZG1 par les couples d'émission-réception 10/11 et 12/13 ont des intensités qui sont sensiblement identiques dans le cas de la figure 2b, celle du couple 10/11 est inférieure à celle du 12/13 dans le cas de la figure 2c, et supérieure à cette dernière dans le cas de la figure 2d. Lorsque l'excentricité β de l'axe optique D1 varie continûment, les intensités mesurées pour les parties de rayonnement qui pénètrent respectivement par les sections d'entrée 11 et 13 varient aussi continûment, en sens opposés. Les couples d'émission-réception 10/11 et 12/13 permettent donc de déterminer la position angulaire de l'axe optique D1 de l'oeil 100 dans un plan horizontal lorsque la tête du porteur est verticale.

Eventuellement, le porteur peut être équipé de lentilles de contact qui améliorent la sensibilité de la détection de la position de chaque oeil. En effet, une telle lentille de contact peut recouvrir l'iris de l'oeil correspondant en restant centrée sur son axe optique, quelque soit la position en rotation de l'oeil. Elle peut alors être conçue pour adapter la valeur du coefficient d'intensité de réflexion apparent de l'iris, afin d'augmenter le contraste de réflexion entre l'iris et la sclérotique.

Une unité calcul est associée à la paire de lunettes. Elle peut être incorporée dans l'une des branches de la monture 3, par exemple. Une telle unité de calcul est adaptée pour déterminer la position angulaire de l'axe optique D1 à partir d'intensités qui sont mesurées pour les parties de rayonnement réfléchies. Cette détermination de la position angulaire de l'axe optique D1 peut être effectuée par calcul. Alternativement, l'unité de calcul peut déterminer la position angulaire de l'axe optique D1 en lisant une table enregistrée qui indique cette position en fonction de valeurs d'intensité mesurées pour les différents chemins de rayonnement, servant d'entrées dans cette table.

Il est entendu que le verre gauche 2 présente une configuration qui est analogue à celle du verre droit 1, en étant symétrique de celui-ci par rapport au plan médian du visage du porteur. L'unité de calcul permet alors de déterminer la position angulaire de l'axe optique D2 de l'oeil gauche 200 du porteur, à des instants qui sont identiques à ceux des positions déterminées pour l'axe optique D1 de l'oeil droit 100. La distance d'observation D ainsi que, éventuellement la position du centre de convergence C des deux yeux et/ou la direction du regard D0, est alors obtenue.

Ainsi, comme il vient d'être décrit, l'unité de calcul peut être adaptée pour déterminer d'abord une position angulaire de l'axe optique de chaque oeil à partir des intensités qui sont mesurées simultanément pour le verre situé devant cet oeil, et pour les parties de rayonnement réfléchies dans certaines des zones oculaires où se déplacent les portions droite et gauche de la limite entre la sclérotique et l'iris de l'oeil. L'unité de calcul est alors aussi adaptée pour déterminer ensuite la convergence des yeux du porteur à partir des positions angulaires respectives des axes optiques des deux yeux qui ont été déterminées pour un même instant. Toutefois, un tel fonctionnement de l'unité de calcul en deux étapes successives n'est pas indispensable. La convergence des yeux du porteur avec, éventuellement, la position angulaire de la direction D0 et la distance d'observation D, peut (peuvent) être déduite(s) en une seule étape à partir d'une (de) combinaison(s) adéquate(s) des intensités qui sont mesurées simultanément pour les parties de rayonnement réfléchies dans les zones latérales des deux yeux, où se déplacent les portions droites et gauches des limbes L correspondants.

Chaque verre est en outre prévu pour déterminer au moins deux paires de chemins de rayonnement, qui comprennent des réflexions sur l'oeil du porteur dans des zones latérales de celui-ci qui sont situées à une même première hauteur α₁ pour les chemins d'une même première paire, et à une même seconde hauteur α₂ pour les chemins d'une même seconde paire. Les figures 2a à 2d montrent la mise en oeuvre de ce perfectionnement lorsque chaque chemin est déterminé par un couple d'émission-réception séparé de ceux des autres chemins. Ainsi, la section de sortie 10 avec la section d'entrée 11 d'une part, et la section de sortie 12 avec la section d'entrée 13 d'autre part, déterminent une première paire de chemins de rayonnement qui sont situés à la valeur de hauteur α₁. De même, la section de sortie 20 avec la section d'entrée 21 d'une part, et la section de sortie 22 avec la section d'entrée 23 d'autre part, déterminent une seconde paire de chemins de rayonnement qui sont situés à la valeur de hauteur α₂. Le couple d'émission-réception 20/21 détermine un chemin optique supplémentaire qui comprend un point de réflexion sur l'oeil 100 qui est situé dans une zone latérale droite ZD2 de celui-ci, à la hauteur α₂ en dessous de la zone ZD1. De même, le couple d'émission-réception 22/23 détermine un autre chemin optique supplémentaire qui comprend un point de réflexion sur l'oeil 100 qui est situé dans une zone latérale gauche ZG2 de celui-ci, à la hauteur α₂ en dessous de la zone ZG1. Dans ce cas, les hauteurs des zones oculaires qui sont associées aux deux paires de chemins de rayonnement peuvent avoir différence qui est comprise entre 10° et 45° en valeur absolue. Ainsi, l'une de ces valeurs de hauteur, par exemple α₁, peut correspondre à l'observation d'un objet qui est sensiblement situé à hauteur d'yeux pour le porteur. L'autre valeur de hauteur, α₂, peut correspondre à l'observation d'un objet qui est situé dans la partie inférieure du champ de vision du porteur. L'unité de calcul peut alors être adaptée pour sélectionner l'une des valeurs de hauteur de zones oculaires pour caractériser la convergence des yeux du porteur. Une telle sélection peut être effectuée, notamment, en fonction d'une valeur d'un rapport signal/bruit de certaines au moins des intensités qui sont mesurées. De cette façon, les chemins de rayonnement qui peuvent être occultés par un abaissement des paupières du porteur ne sont pas pris en compte pour déterminer la convergence de ses yeux.

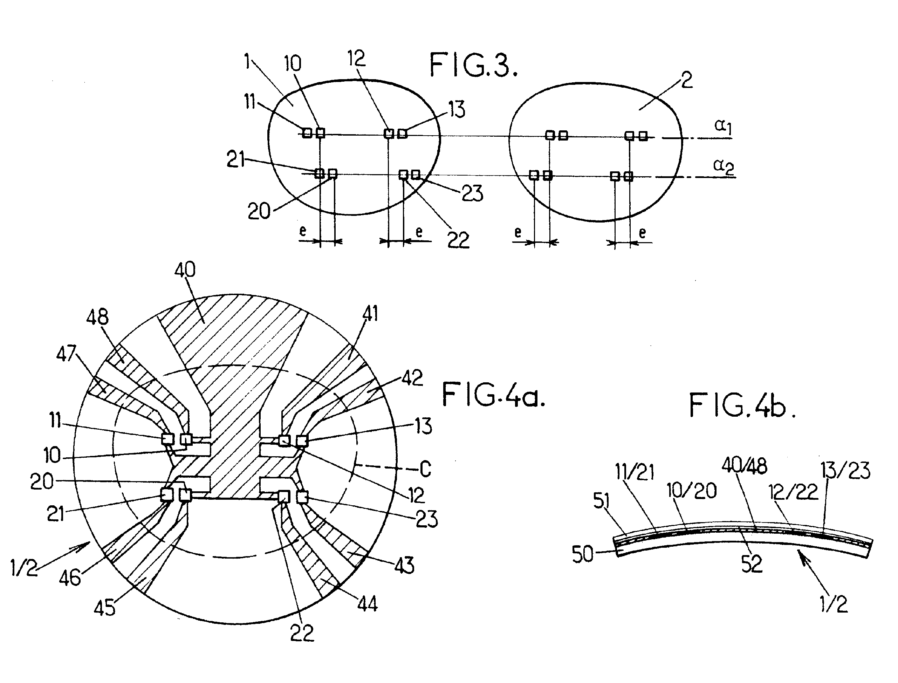

La figure 3 illustre un perfectionnement de l'invention, suivant lequel les couples d'émission-réception qui correspondent à l'une des valeurs de hauteur sont décalés latéralement par rapport aux couples d'émission-réception qui correspondent à l'autre valeur de hauteur. Ainsi, les couples d'émission-réception 20/21 et 22/23 qui définissent des réflexions sur l'oeil à la hauteur α₂ sont décalés de la distance e vers le côté nasal par rapport aux couples d'émission-réception 10/11 et 12/13 qui définissent des réflexions sur l'oeil à la hauteur α₁. Lorsque les verres 1 et 2 sont du type à addition progressive de puissance optique, couramment désignés par verres progressifs, les valeurs de hauteur α₁ et α₂ peuvent correspondre respectivement aux hauteurs dans chaque verre des directions de vision de loin et de près. Le décalage e peut alors être sensiblement égal au décalage qui résulte de la convergence variable des yeux entre les conditions de vision de loin et de près, et qui est couramment appelé inset. Dans ce cas, le décalage e peut être compris entre 4 et 6,5 mm. Plus généralement, il peut être compris entre 0 et 7 mm.

Selon un premier type de réalisations de l'invention, chaque source du rayonnement peut être une diode à émission lumineuse qui fonctionne à une longueur d'onde comprise entre 900 nm et 1200 nm. Dans ce cas, chaque section de sortie de rayonnement correspond à une portion de surface d'émission de cette diode. Alternativement, chaque source du rayonnement peut être une source VCSEL, pour «Vertical Cavity Surface Emitting Laser».

Parallèlement, chaque détecteur peut être une photodiode ou un phototransistor. Chaque section d'entrée de rayonnement correspond alors à une portion de surface photosensible de la photodiode ou du phototransistor.

Selon une combinaison particulièrement avantageuse, chaque source du rayonnement et chaque détecteur sont des composants micro-optoélectroniques à base de matériau semiconducteur, qui sont intégrés dans chaque verre. De cette façon, chaque verre est un élément autonome qui est adapté pour mettre en oeuvre l'invention, avec des coûts de fabrication et d'assemblage dans la monture qui peuvent être très faibles. Dans ce cas, chaque verre comprend en outre des pistes conductrices transparentes qui relient électriquement des bornes de chaque source et de chaque détecteur. Ces pistes relient les sources et détecteurs à l'unité de calcul qui peut être externe au verre de lunettes. De préférence, les pistes conductrices sont dirigées radialement dans une partie périphérique du verre. Grâce à une telle disposition des pistes, le verre peut être détouré selon un contour de son logement dans la monture 3, tout en repérant simplement les parties de ce contour dans lesquelles débouchent séparément les pistes conductrices. Par exemple, les pistes conductrices occupent des secteurs angulaires séparés dans la partie périphérique du verre. Conformément aux figures 4a et 4b, chaque verre 1, 2 peut comprendre une partie de base 50 et une partie d'encapsulation 51 qui sont solidaires, et chaque source du rayonnement, chaque détecteur et chaque piste conductrice peuvent être disposés entre les parties 50 et 51. Par exemple, la piste 40 peut constituer une borne de masse qui est commune à toutes les sources de rayonnement et détecteurs du verre, et les pistes 41 à 48 relient respectivement une autre borne de chacune des sources 10, 12, 20, 22 et de chacun des détecteurs 11, 13, 21, 23. Ces pistes, sources et détecteurs peuvent être formés sur la partie de base 50 et être enrobés dans une couche de colle transparente 52 qui assure la liaison mécanique avec la partie d'encapsulation 51. Les sources et détecteurs du rayonnement ont avantageusement des dimensions respectives qui sont très petites, de façon à ne pas gêner la vision du porteur et être invisibles. En outre, les pistes 40-48 peuvent être constituées de tout matériau qui est à la fois transparent et conducteur électriquement, tel que l'oxyde d'indium dopé à l'étain, ou ITO pour «indium-tin oxide» en anglais. Alternativement, chaque piste 40-48 peut être remplacée par un fil conducteur très fin, qui est disposé radialement dans la partie périphérique du verre. Sur la figure 4a, C désigne un contour de détourage qui est indiqué à titre d'exemple. Le contour C coupe chacune des pistes 40-48 dans une partie de celle-ci qui est dirigée radialement.

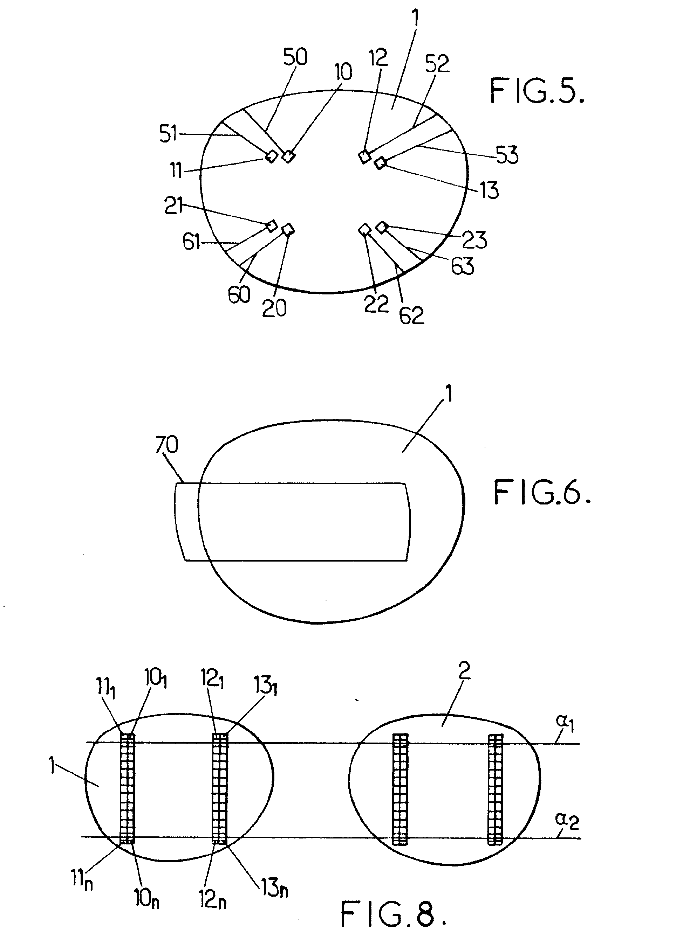

Selon un deuxième type de réalisations de l'invention, chaque source de rayonnement peut être située à l'extérieur du verre correspondant, et être couplée optiquement à une des sections de sortie par un premier guide optique, par exemple par une fibre optique. De même, chaque détecteur de rayonnement peut aussi être situé à l'extérieur du verre, et aussi être couplé optiquement à une des sections d'entrée de rayonnement par un second guide optique, qui peut être distinct ou confondu avec l'un des premiers guides optiques. Selon un mode de fabrication particulier du verre, chaque premier ou second guide optique peut être incorporé au verre lors du moulage de celui-ci, en étant disposé dans un moule d'injection avant que le matériau constitutif du verre soit introduit dans le moule. Eventuellement, chaque section de sortie ou d'entrée du rayonnement peut être pourvue d'un micro-prisme qui est intégré au verre, et qui détermine la direction d'émission du rayonnement vers une zone latérale de l'oeil, ou la direction de collecte de la partie réfléchie du rayonnement. Autrement dit, chaque section de sortie ou d'entrée du rayonnement est formée par une face d'un microprisme situé à l'extrémité d'un guide optique. Sur la figure 5, les références 50-53 et 60-63 désignent des fibres optiques qui relient respectivement les sections de sortie et d'entrée 10-13 et 20-23. Pour la même raison que précédemment à propos des pistes conductrices, les fibres optiques 50-53 et 60-63 sont de préférence disposées radialement dans le verre 1, pour que leurs positions au niveau du contour de détourage C soient facilement connues.

Selon un troisième type de réalisations de l'invention, la paire de lunettes peut comprendre en outre, pour chaque verre, un guide optique qui forme une image d'une partie de l'oeil à l'extérieur de ce verre. Sur la figure 6, la référence 70 désigne un tel guide optique, dont des réalisations sont connues de l'Homme du métier et ne sont pas reprises ici. Selon une configuration avantageuse, le guide optique 70 pénètre dans le verre 1 à partir du bord temporal de celui-ci, au niveau de la branche de la monture 3, pour ne pas réduire le confort d'utilisation et l'esthétisme du verre. Eventuellement, le guide optique peut aussi amener le rayonnement d'une source qui est externe au verre, pour produire une intensité lumineuse qui soit suffisante pour la détection de l'image de l'oeil. Il délivre alors le rayonnement de la source à travers au moins quatre zones du guide optique 70, qui forment les sections de sortie du rayonnement. Avantageusement, une face active du guide optique 70 qui est située en face de l'oeil est continue entre les sections de sortie. Les détecteurs sont alors répartis dans des zones de l'image de l'oeil où se déplacent lors des mouvements oculaires les portions droite et gauche de la limite entre la sclérotique et l'iris de l'oeil, qui sont imagées par le guide optique.

Les figures 7a à 7d illustrent un autre mode de réalisation d'un verre selon l'invention, dans lequel plusieurs chemins optiques qui comprennent des points de réflexion respectifs dans les zones latérales ZD1, ZD2, ZG1 et ZG2 de l'oeil, ont en commun une même section d'entrée du rayonnement. Sur ces figures, les références 30 et 32 désignent les sections de sortie du rayonnement qui sont situées à une même première hauteur dans le verre, les références 40 et 42 désignent les sections de sortie du rayonnement qui sont situées à une même seconde hauteur dans le verre, et la référence 31 désigne la section d'entrée qui est commune aux quatre sections de sortie. Le premier chemin de rayonnement est donc issu de la section de sortie 30, comprend une réflexion dans la zone oculaire latérale ZD1 où se déplace la partie supérieure droite du limbe L, et aboutit à la section d'entrée 31. Le deuxième chemin de rayonnement est issu de la section de sortie 32, comprend une réflexion dans la zone oculaire latérale ZG1 où se déplace la partie supérieure gauche du limbe L, et aboutit aussi à la section d'entrée 31. De même, le troisième chemin de rayonnement est issu de la section de sortie 40, comprend une réflexion dans la zone oculaire latérale ZD2 où se déplace la partie inférieure droite du limbe L, et aboutit aussi à la section d'entrée 31. Enfin, le quatrième chemin de rayonnement est issu de la section de sortie 42, comprend une réflexion dans une zone oculaire latérale ZG2 où se déplace la partie inférieure gauche du limbe L, et aboutit aussi à la section d'entrée 31. Dans ce cas, les sections de sortie 30, 32, 40 et 42 peuvent chacune être alimentées en rayonnement selon quatre modulations temporelles différentes, et le détecteur qui est connecté optiquement à la section d'entrée 31 est contrôlé pour réaliser une détection synchrone. Une telle détection synchrone permet de séparer les parties de rayonnement qui suivent l'un ou l'autre des quatre chemins qui passe par l'une des zones oculaires, bien que les quatre chemins partagent la même section d'entrée et le même détecteur. D'autres moyens peuvent être utilisés alternativement pour séparer les parties de rayonnement qui suivent l'un ou l'autre des chemins. Par exemple, des longueurs d'onde de rayonnement ou des polarisations qui sont différentes peuvent être utilisées pour chacun des chemins, et un filtrage est mis en oeuvre pour séparer les parties de rayonnement qui sont collectées par la section d'entrée commune en provenance de chaque zone oculaire.

Les figures 7c et 7d montrent des positions différentes du limbe L de l'oeil 100, par rapport aux zones oculaires latérales qui contiennent les réflexions des quatre chemins de rayonnement. Les intensités des parties réfléchies des rayonnements qui ont suivi l'un ou l'autre des quatre chemins varient d'une même façon que celle déjà décrite en relation avec les figures 2c et 2d.

Dans encore un autre mode de réalisation d'un verre selon l'invention, plusieurs chemins qui comprennent des points de réflexion respectifs dans les zones latérales de l'oeil, peuvent avoir en commun une même section de sortie du rayonnement, et éventuellement aussi une même source du rayonnement. Ils aboutissent alors à des sections d'entrée de rayonnement qui sont différentes. Un tel mode de réalisation peut être déduit simplement du précédent et n'est pas décrit en détail pour cette raison.

Les deux derniers modes de réalisation de l'invention qui ont été décrits ci-dessus permettent de simplifier la réalisation des verres de lunettes, puisque ceux-ci comprennent alors un nombre de composants qui est réduit.

Enfin, la figure 8 illustre encore un autre mode de réalisation de l'invention, qui est déduit de celui des figures 2a à 2d. Les deux sections de sortie de rayonnement et les deux sections d'entrée de rayonnement sont remplacées dans les parties droite et gauche de chaque verre de lunettes, respectivement par des colonnes de sections de sortie et des colonnes de sections d'entrée. Ainsi, la partie droite du verre 1 contient une colonne de sections de sorties 10₁,..., 10ₙ, où n est un entier naturel supérieur à 2, et une colonne de sections d'entrée 11₁,..., 11ₙ. Ces deux colonnes sont verticales par rapport à la position d'utilisation du verre 1 par le porteur, parallèles, de préférence rapprochées l'une de l'autre, avec les sections de sortie et d'entrée qui sont décalées progressivement vers le bas du verre avec des décréments de hauteur qui sont identiques. Toutefois, il n'est pas nécessaire que les sections à l'intérieur d'une même colonne soient contigües, de sorte qu'elles peuvent être séparées deux à deux par des intervalles. La partie gauche du verre 1 contient une colonne de sections de sortie 12₁,..., 12ₙ et une colonne de sections d'entrée 13₁,..., 13ₙ qui peuvent être analogues à celles de la partie droite du même verre 1. Le verre 2 possède alors une structure symétrique de celle du verre 1. Eventuellement, ces colonnes de sections de sortie ou d'entrée peuvent être constituées directement pas des colonnes de sources, par exemple du type diode à émission lumineuse infrarouge, ou par des colonnes de détecteurs, par exemple du type photodiode ou phototransistor.

L'utilisation de telles colonnes de sources ou de détecteurs est particulièrement avantageuse pour obtenir une caractérisation de la convergence des yeux du porteur, sans nécessiter une consommation d'énergie qui soit trop importante. La source d'énergie qui alimente les sources, les détecteurs et l'unité de calcul peut alors être de capacité réduite. En effet, les sources et détecteurs qui correspondent à des chemins de rayonnement ayant des réflexions sur l'oeil qui sont situées à une même hauteur de zone oculaire, peuvent être sélectionnées conformément à l'une des stratégies suivantes, alors que les autres sources et détecteurs ne sont pas activés :
/i/ les sources et détecteurs peuvent être activés de façon séquentielle, selon un ordre de balayage qui est prédéterminé ;
/ii/ les sources et détecteurs qui sont activés pour une nouvelle séquence de mesure d'intensités de parties de rayonnement réfléchies sur l'oeil peuvent être sélectionnés en fonction d'au moins une valeur d'un rapport signal/bruit qui a été obtenue lors d'une séquence de mesure antérieure ;
/iii/ ils peuvent être sélectionnés en fonction d'un algorithme prédictif, quant à la hauteur de réflexion sur l'oeil qui est la plus adaptée pour caractériser de nouveau la convergence du regard du porteur, en fonction des hauteurs qui ont déjà été sélectionnées lors de caractérisations antérieures. Cette stratégie peut tenir compte d'une hauteur de la direction de regard qui a été déterminée antérieurement ; et
/iv/ une stratégie composite qui combine les critères de sélection des stratégies /i/ à /iii/ précédentes.

Ces stratégies sont particulièrement adaptées pour éviter que les mouvements de paupières du porteur ne perturbent la caractérisation de la convergence de son regard, réalisée selon l'invention.

L'Homme du métier comprendra que l'invention n'est pas limitée aux modes de réalisation particuliers qui ont été décrits en relation avec les figures. Notamment, les différents types de réalisations de l'invention qui ont été énumérés peuvent être combinés de multiples façons avec des tracés différents des chemins de rayonnement, ces chemins comportant des points de réflexion respectifs dans les zones latérales de l'oeil où se déplacent les portions droite et gauche du limbe. En outre, les positions des sections de sortie et d'entrée du rayonnement à l'intérieur de chaque verre peuvent être modifiées librement, pour former des chemins de rayonnement qui comprennent des points de réflexion sur l'oeil situés à l'intérieur de zones variables. Eventuellement, une même section de passage de rayonnement à travers le verre peut être à la fois une section de sortie de rayonnement et une section d'entrée de rayonnement. Dans ce cas, cette section de passage est sensiblement située le long de la direction qui est perpendiculaire à la surface de l'oeil dans la zone oculaire correspondante.

Notamment, dans un mode de réalisation où les sections de sortie et d'entrée de rayonnement qui appartiennent à un même couple d'émission-réception sont juxtaposées, les positions relatives de la section de sortie et de la section d'entrée peuvent être modifiées en conservant un point de réflexion du chemin de rayonnement correspondant qui reste au même endroit sur l'oeil. Notamment, la position de la section de sortie et celle de la section d'entrée peuvent être échangées.

Divers perfectionnements de l'invention sont maintenant décrits, qui augmentent la fiabilité de la détermination de la convergence des yeux, et/ou qui améliorent le confort et/ou la sécurité du porteur de la paire de lunettes.

Selon un premier de ces perfectionnements, la paire de lunettes peut comprendre en outre des moyens de modulation du rayonnement qui est produit par chaque source, et des moyens de traitement du signal de détection qui est produit par chaque détecteur. Ces moyens sont adaptés pour réaliser une détection synchrone de chaque partie du rayonnement qui est réfléchie sur l'oeil du porteur, basée sur la modulation de la source. Une telle détection synchrone permet en particulier de distinguer le rayonnement qui est mis en oeuvre pour l'invention d'un rayonnement ambiant parasite. En particulier, la modulation peut être sélectionnée pour que la détection synchrone supprime efficacement des contributions du rayonnement ambiant à des fréquences qui sont multiples de 50 Hz (Hertz), de telles contributions étant couramment produites par des systèmes d'éclairage à décharge.

Selon un deuxième perfectionnement, la paire de lunettes peut comprendre en outre des moyens de filtrage d'un signal de mesure qui est produit par chaque détecteur. Ces moyens peuvent être adaptés pour supprimer des mesures qui correspondent à des variations involontaires de la convergence des yeux du porteur. En effet, les variations involontaires de la convergence des yeux du porteur sont en général beaucoup plus rapides que les variations qui résultent de changements volontaires entre des objets qui sont regardés successivement par le porteur. De cette façon, la détermination de la convergence des yeux du porteur peut être limitée à des changements d'attention visuelle dont le porteur est conscient. Ainsi, lorsque l'invention est utilisée pour commander une caractéristique variable des verres, cette caractéristique n'est modifiée que dans une mesure utile par rapport à l'attention visuelle du porteur.

Enfin, selon un troisième perfectionnement, la paire de lunettes peut comprendre encore des moyens de contrôle de chaque source de rayonnement, qui sont adaptés pour activer un fonctionnement intermittent de cette source conformément à un rapport cyclique compris entre 2% et 50%, de préférence inférieur à 10%. La valeur de ce rapport cyclique peut être sélectionnée en fonction de plusieurs critères différents, parmi lesquels on peut citer une limitation de la quantité de rayonnement qui est dirigée vers l'oeil et une limitation de la puissance électrique qui est consommée par un verre de lunettes selon l'invention. En effet, un tel verre est de préférence alimenté en courant électrique à partir d'une pile ou d'une batterie qui est logée dans la monture. Par ailleurs, la source peut être activée pour effectuer deux déterminations successives de la convergence des yeux du porteur qui sont séparées par une durée fixée. Une telle durée d'attente est en général indépendante du rapport cyclique de fonctionnement des sources. Elle peut être déterminée, notamment, en fonction du temps qui est nécessaire à l'unité de calcul pour déterminer la convergence des yeux à partir des signaux de mesure produits par les détecteurs.

## Revendications

1. Paire de lunettes ophtalmiques, comprenant une monture (3) et deux verres (1, 2) maintenus dans la monture pour être situés respectivement devant les yeux (100, 200) d'un porteur des dites lunettes, et permettant une vision distincte pour le porteur à travers chaque verre, la paire de lunettes comprenant pour chaque verre :
- au moins une source d'un rayonnement sélectionnée pour que ledit rayonnement soit réfléchi par la sclérotique (S) et par l'iris (I) de chaque oeil du porteur conformément à des coefficients d'intensité de réflexion différents respectivement pour la sclérotique et pour l'iris ;
- au moins un détecteur disposé pour mesurer des intensités respectives de parties du rayonnement produit par la source, réfléchies respectivement dans des zones oculaires (ZD1, ZG1, ZD2, ZG2) dans chacune desquelles se déplace une portion droite ou gauche d'une limite (L) entre la sclérotique et l'iris de l'oeil situé derrière ledit verre lors de mouvements oculaires ; et
- au moins quatre sections de sortie ou d'entrée de rayonnement disposées dans le verre, chaque section de sortie (10, 12, 20, 22) étant disposée pour diriger une partie du rayonnement produit par la source vers au moins une des dites zones oculaires, et chaque section d'entrée (11, 13, 21, 23) étant disposée pour collecter une partie du rayonnement réfléchie dans l'une des dites zones oculaires ;
de façon à former au moins quatre chemins de rayonnement comportant chacun une réflexion sur l'oeil du porteur situé derrière ledit verre, dans l'une des zones oculaires où se déplacent les portions droite et gauche, respectivement pour l'un et l'autre des dits chemins, de la limite entre la sclérotique et l'iris dudit oeil;
la paire de lunettes comprenant en outre des moyens pour séparer les parties de rayonnement transmises entre ladite au moins une source de rayonnement et ledit au moins un détecteur respectivement par des chemins de rayonnement différents ; et
la paire de lunettes comprenant en outre une unité de calcul adaptée pour caractériser une convergence des yeux du porteur vers un point d'observation commun (C), en fonction des intensités mesurées simultanément pour les deux verres ;
pour chaque verre (1, 2), les dits au moins une source, au moins un détecteur et au moins quatre zones de sortie (10, 12, 20, 22) ou d'entrée (11, 13, 21, 23) de rayonnement étant disposés de façon à former au moins deux paires de chemins distincts pour le rayonnement, avec deux premières sections de sortie de rayonnement (10, 12) qui sont disposées dans chaque verre à une même première hauteur (α₁), et deux secondes sections de sortie de rayonnement (20, 22) qui sont disposées dans chaque verre à une même seconde hauteur (α₂) différente de ladite première hauteur,
de façon que les zones oculaires (ZD1, ZG1, ZD2, ZG2) où se déplacent les portions droite et gauche, respectivement pour l'un et l'autre des chemins de chaque paire, de la limite (L) entre la sclérotique et l'iris de l'oeil du porteur situé derrière ledit verre, sont situées à une même hauteur (α₁, α₂) de zones oculaires associée à ladite paire, et les deux paires de chemins étant associées à des hauteurs respectives de zones oculaires distinctes et communes aux deux verres ;
et l'unité de calcul étant adaptée pour caractériser la convergence des yeux du porteur en fonction des intensités mesurées simultanément pour les parties de rayonnement transmises par les chemins associés à une même des hauteurs de zones oculaires, sélectionnée par ladite unité de calcul pour les deux verres.

2. Paire de lunettes selon la revendication 1, dans laquelle l'unité de calcul est adaptée pour sélectionner une des hauteurs (α₁, α₂) de zones oculaires (ZD1, ZG1, ZD2, ZG2) pour caractériser la convergence des yeux du porteur, en fonction d'une valeur d'un rapport signal/bruit de certaines au moins des intensités mesurées.

3. Paire de lunettes selon la revendication 1 ou 2, dans laquelle l'unité de calcul est adaptée pour déterminer d'abord une position angulaire d'un axe optique (D1, D2) de chaque oeil (100, 200) à partir des intensités mesurées simultanément pour le verre (1, 2) situé devant ledit oeil, et pour des parties de rayonnement réfléchies dans certaines des zones oculaires (ZD1, ZG1, ZD2, ZG2) où se déplacent les portions droite et gauche de la limite (L) entre la sclérotique (S) et l'iris (I) dudit oeil;
et l'unité de calcul est en outre adaptée pour déterminer ensuite la convergence des yeux du porteur à partir des positions angulaires respectives des axes optiques des deux yeux déterminées pour un même instant.

4. Paire de lunettes selon l'une quelconque des revendications 1 à 3, comprenant autant de sections de sortie de rayonnement (10, 12, 20, 22) que de sections d'entrée de rayonnement (11, 13, 21, 23) pour chaque verre, chacune des dites sections de sortie étant juxtaposée dans le verre avec l'une des dites sections d'entrée pour former un couple séparé d'émission-réception de rayonnement, chaque couple d'émission-réception étant disposé devant une des zones oculaires (ZD1, ZG1, ZD2, ZG2) où se déplace l'une des portions droite et gauche de la limite (L) entre la sclérotique (S) et l'iris (I) de l'oeil du porteur situé derrière ledit verre lors des mouvements oculaires.

5. Paire de lunettes selon l'une quelconque des revendications 1 à 4, dans laquelle, pour chaque verre (1, 2), les sections de sortie de rayonnement sont disposées sous forme de deux colonnes situées respectivement dans des parties droite et gauche dudit verre, chaque section de sortie de la colonne (10₁,.... 10ₙ) située dans la partie droite du verre appartenant à un chemin de rayonnement comportant une réflexion sur l'oeil (100) du porteur dans la zone oculaire où se déplace la portion droite de la limite (L) entre la sclérotique (S) et l'iris (1) dudit oeil, et chaque section de sortie de la colonne (12₁,..., 12ₙ) située dans la partie gauche du verre appartenant à un chemin de rayonnement comportant une réflexion sur l'oeil du porteur dans la zone oculaire où se déplace la portion gauche de ladite limite entre la sclérotique et l'iris de l'oeil.

6. Paire de lunettes selon l'une quelconque des revendications 1 à 4, dans laquelle ladite au moins une source, les sections de sortie, au moins une section d'entrée et ledit au moins un détecteur qui sont associés à l'un au moins des verres, sont disposés de sorte qu'au moins deux chemins différents de rayonnement traversant des sections de sortie différentes (30, 32, 40, 42) pénètrent par une même section d'entrée (31) commune aux dits chemins, et les moyens pour séparer les parties de rayonnement en fonction des chemins de rayonnement sont adaptés pour synchroniser des mesures réalisées par ledit au moins un détecteur conformément à des modulations différentes appliquées aux rayonnements transmis par lesdits chemins différents.

7. Paire de lunettes selon l'une quelconque des revendications 1 à 6, dans laquelle chaque source du rayonnement et chaque détecteur sont des composants micro-optoélectroniques à base de matériau semiconducteur, intégrés dans le verre correspondant, et ledit verre comprend en outre des pistes conductrices transparentes (40-48) reliant électriquement des bornes de chaque détecteur et de chaque source à l'unité de calcul, et dirigées radialement dans une partie périphérique du verre.

8. Paire de lunettes selon l'une quelconque des revendications 1 à 6, dans laquelle chaque source de rayonnement est située à l'extérieur du verre correspondant, et est couplée optiquement à une des sections de sortie (10, 12, 20, 22) par un premier guide optique (50, 52, 60, 62), et chaque détecteur est situé à l'extérieur du verre correspondant, et est couplé optiquement à une des sections d'entrée (11, 13, 21, 23) par un second guide optique (51, 53, 61, 63).

9. Paire de lunettes selon l'une quelconque des revendications 1 à 6, comprenant en outre, pour chaque verre (1, 2), un guide optique (70) qui forme une image d'une partie de l'oeil (100, 200) à l'extérieur dudit verre, et dans laquelle les détecteurs sont répartis dans des zones de ladite image où se déplacent les portions droite et gauche de la limite (L) entre la sclérotique (S) et l'iris (I) dudit oeil qui sont imagées lors des mouvements oculaires.

10. Paire de lunettes selon l'une quelconque des revendications précédentes, comprenant en outre des moyens de variation d'une caractéristique de l'un au moins des verres, en fonction d'un résultat de la convergence des yeux du porteur **caractérisée par** l'unité de calcul.

11. Verre de lunettes (1, 2) comprenant :
- au moins une source d'un rayonnement (10, 12, 20, 22), chaque source étant intégrée audit verre et sélectionnée pour que ledit rayonnement soit réfléchi par une sclérotique (S) et par un iris (I) d'un oeil (100, 200) d'un porteur du verre, conformément à des coefficients d'intensité de réflexion différents respectivement pour la sclérotique et pour l'iris ;
- au moins un détecteur dudit rayonnement (11, 13, 21, 23), chaque détecteur étant intégré audit verre et disposé pour mesurer une intensité d'une partie du rayonnement produit par l'une des sources et réfléchie par l'oeil dans une zone oculaire (ZD1, ZG1, ZD2, ZG2) dans laquelle se déplace une portion droite ou gauche d'une limite (L) entre la sclérotique et l'iris dudit oeil lors de mouvements oculaires ;
- des pistes conductrices transparentes (40-48) intégrées audit verre, reliant électriquement des bornes de chaque détecteur et de chaque source, et dirigées radialement dans une partie périphérique du verre ; et
- au moins quatre sections de sortie ou d'entrée de rayonnement disposées dans le verre, chaque section de sortie (10, 12, 20, 22) étant disposée pour diriger une partie du rayonnement produit par la source vers au moins une des dites zones oculaires, et chaque section d'entrée (11, 13, 21, 23) étant disposée pour collecter une partie du rayonnement réfléchie dans l'une des dites zones oculaires ;
de façon à former au moins deux chemins de rayonnement comportant chacun une réflexion sur l'oeil du porteur situé derrière ledit verre, dans l'une des zones oculaires où se déplacent les portions droite et gauche, respectivement pour l'un et l'autre des dits chemins, de la limite entre la sclérotique et l'iris dudit oeil;
avec deux premières sections de sortie de rayonnement (10, 12) qui sont disposées à une même première hauteur (α₁) dans le verre, et deux secondes sections de sortie de rayonnement (20, 22) qui sont disposées dans ledit verre à une même seconde hauteur (α₂) différente de ladite première hauteur.

12. Verre de lunettes selon la revendication 11, comprenant autant de sources de rayonnement (10, 12, 20, 22) que de détecteurs (11, 13, 21, 23), chacune des sources étant juxtaposée dans le verre avec l'un des détecteurs pour former un couple séparé d'émission-réception de rayonnement, chaque couple d'émission-réception étant disposé devant une des zones oculaires (ZD1, ZG1, ZD2, ZG2) où se déplace l'une des portions droite et gauche de la limite (L) entre la sclérotique (S) et l'iris (I) de l'oeil du porteur lors des mouvements oculaires.

13. Verre de lunettes selon la revendication 11 ou 12, dans lequel les sources sont disposées sous forme de deux colonnes situées respectivement dans des parties droite et gauche dudit verre, chaque source de la colonne (10₁,..., 10ₙ) située dans la partie droite du verre (1) appartenant à un chemin de rayonnement comportant une réflexion sur l'oeil (100) du porteur dans la zone oculaire où se déplace la portion droite de la limite (L) entre la sclérotique (S) et l'iris (I) dudit oeil, et chaque source de la colonne (12₁,.... 12ₙ) située dans la partie gauche du verre appartenant à un chemin de rayonnement comportant une réflexion sur l'oeil du porteur dans la zone oculaire où se déplace la portion gauche de ladite limite entre la sclérotique et l'iris de l'oeil.

14. Verre de lunettes selon l'une quelconque des revendications 11 à 13, dans lequel chaque source du rayonnement (10, 12, 20, 22) est une diode à émission lumineuse fonctionnant à une longueur d'onde comprise entre 900 nm et 1200 nm.

15. Verre de lunettes selon l'une quelconque des revendications 11 à 14, dans lequel chaque détecteur (11, 13, 21, 23) est une photodiode ou un phototransistor.

16. Verre de lunettes selon l'une quelconque des revendications 11 à 15, comprenant en outre une partie de base du verre (50) et une partie d'encapsulation du verre (51), les dites parties de base et d'encapsulation étant solidaires, et chaque source du rayonnement (10, 12, 20, 22), chaque détecteur (11, 13, 21, 23) et chaque piste conductrice (40-48) dudit verre étant disposés entre les dites parties de base et d'encapsulation.

17. Verre de lunettes selon l'une quelconque des revendications 11 à 16, comprenant en outre des moyens de variation d'une caractéristique dudit verre.

## Patentansprüche

1. Ophthalmische Brille, die eine Fassung (3) und zwei Gläser (1, 2), die in der Fassung gehalten werden, damit sie sich vor den jeweiligen Augen (100, 200) eines Trägers der Brille befinden, und eine unterschiedliche Sicht für den Träger durch jedes Glas ermöglichen, umfasst, wobei die Brille für jedes Glas Folgendes umfasst:
- wenigstens eine ausgewählte Strahlungsquelle, damit die Strahlung durch die Lederhaut (S) und durch die Iris (I) jedes Auges des Trägers entsprechend den verschiedenen Reflexionsintensitätskoeffizienten für die Lederhaut bzw. für die Iris reflektiert wird;
- wenigstens einen Detektor, der dazu ausgelegt ist, die jeweiligen Intensitäten der durch die Quelle erzeugten Strahlungsanteile, die jeweils in Okularzonen (ZD1, ZG1, ZD2, ZG2) reflektiert werden, zu messen, wobei sich in jeder von ihnen ein rechter oder linker Abschnitt einer Grenze (L) zwischen der Lederhaut und der Iris des Auges, der sich hinter dem Glas befindet, bei Okularbewegungen verlagert; und
- wenigstens vier Austritts- oder Eintrittsabschnitte für die Strahlung, die in dem Glas angeordnet sind, wobei jeder Austrittsabschnitt (10, 12, 20, 22) so angeordnet ist, dass ein Teil der durch die Quelle erzeugten Strahlung wenigstens zu einer der Okularzonen gelenkt wird, und jeder Eintrittsabschnitt (11, 13, 21, 23) dafür ausgelegt ist, einen Teil der Strahlung, die in einer der Okularzonen reflektiert wird, zu sammeln;
derart, dass wenigstens vier Strahlungswege gebildet werden, die jeweils eine Reflexion auf dem Auge des Trägers, das sich hinter dem Glas befindet, in einer der Okularzonen, wo sich für den einen und den anderen der Wege die rechten bzw. linken Abschnitte der Grenze zwischen der Lederhaut und der Iris des Auges verlagern, aufweisen;
wobei die Brille außerdem Mittel umfasst, um die Strahlungsanteile, die zwischen der wenigstens einen Strahlungsquelle und dem wenigstens einen Detektor auf den jeweils verschiedenen Strahlungswegen durchgelassen werden, zu trennen; und
wobei die Brille außerdem eine Recheneinheit umfasst, die dafür ausgelegt ist, eine Konvergenz der Augen des Trägers zu einem gemeinsamen Beobachtungspunkt (C) als Funktion der gleichzeitig gemessenen Intensitäten für die zwei Gläser zu kennzeichnen;
wobei für jedes Glas (1, 2) die wenigstens eine Quelle, wenigstens ein Detektor und wenigstens vier Austrittszonen (10, 12, 20, 22) oder Eintrittszonen (11, 13, 21, 23) für die Strahlung in der Weise angeordnet sind, dass wenigstens zwei Paare unterschiedlicher Wege für die Strahlung gebildet werden, mit zwei ersten Strahlungsaustrittsabschnitten (10, 12), die in jedem Glas auf derselben ersten Höhe (α₁) angeordnet sind, und zwei zweiten Strahlungsaustrittsabschnitten (20, 22), die in jedem Glas auf derselben zweiten Höhe (α₂), die von der ersten Höhe verschieden ist, angeordnet sind,
derart, dass die Okularzonen (ZD1, ZG1, ZD2, ZG2), wo sich die rechten und linken Abschnitte für den einen und den anderen der Wege jedes Paars der Grenze (L) zwischen der Lederhaut und der Iris des Auges des Trägers, das sich hinter dem Glas befindet, verlagern, sich auf derselben Höhe (α₁, α₂) der Okularzonen, die dem Paar zugeordnet ist, befinden und die zwei Paare von Wegen jeweiligen Höhen verschiedener Okularzonen, die beiden Gläsern gemeinsam sind, zugeordnet sind;
und wobei die Recheneinheit dafür ausgelegt ist, die Konvergenz der Augen des Trägers als Funktion der gleichzeitig gemessenen Intensitäten für die Strahlungsanteile, die von den Wegen, die derselben Höhe der Okularzonen zugeordnet sind, die für die Recheneinheit für die zwei Gläser gewählt wird, durchgelassen werden, zu kennzeichnen.

2. Brille nach Anspruch 1, wobei die Recheneinheit dafür ausgelegt ist, eine der Höhen (α₁, α₂) von Okularzonen (ZD1, ZG1, ZD2, ZG2) zu wählen, um die Konvergenz der Augen des Trägers als Funktion eines Werts des Rauschabstands wenigstens von Bestimmten der gemessenen Intensitäten zu kennzeichnen.

3. Brille nach Anspruch 1 oder 2, wobei die Recheneinheit dafür ausgelegt ist, zunächst eine Winkelposition einer optischen Achse (D1, D2) jedes Auges (100, 200) anhand der gleichzeitig gemessenen Intensitäten für das Glas (1, 2), das sich vor dem Auge befindet, und für Strahlungsanteile, die in bestimmten der Okularzonen (ZD1, ZG1, ZD2, ZG2), wo sich die rechten und linken Abschnitte der Grenze (L) zwischen der Lederhaut (S) und der Iris (I) des Auges verlagern, reflektiert werden, zu bestimmen;
und wobei die Recheneinheit außerdem dafür ausgelegt ist, anschließend die Konvergenz der Augen des Trägers anhand der jeweiligen Winkelpositionen der optischen Achsen der zwei Augen, die für denselben Zeitpunkt bestimmt werden, zu bestimmen.

4. Brille nach einem der Ansprüche 1 bis 3, die ebenso viele Strahlungsaustrittsabschnitte (10, 12, 20, 22) wie Strahlungseintrittsabschnitte (11, 13, 21, 23) für jedes Glas umfasst, wobei jeder der Austrittsabschnitte in dem Glas neben einem der Eintrittsabschnitte liegt, um ein getrenntes Strahlungsaussende/Strahlungsempfangs-Paar zu bilden, wobei jedes Aussende/Empfangs-Paar vor einer der Okularzonen (ZD1, ZG1, ZD2, ZG2), wo sich einer der rechten und linken Abschnitte der Grenze (L) zwischen der Lederhaut (S) und der Iris (I) des Auges des Trägers, das sich hinter dem Glas befindet, während der Okularbewegungen verlagert, angeordnet ist.

5. Brille nach einem der Ansprüche 1 bis 4, wobei für jedes Glas (1, 2) die Strahlungsaustrittsabschnitte in Form von zwei Säulen angeordnet sind, die sich im rechten bzw. im linken Teil des Glases befinden, wobei jeder Austrittsabschnitt der Säule (10₁, ..., 10ₙ), die sich im rechten Teil des Glases befindet, zu einem Strahlungsweg gehört, der eine Reflexion auf dem Auge (100) des Trägers in der Okularzone enthält, wo sich der rechte Abschnitt der Grenze (L) zwischen der Lederhaut (S) und der Iris (I) des Auges verlagert, und jeder Austrittsabschnitt der Säule (12₁, ..., 12ₙ), der sich im linken Teil des Glases befindet, der zu einem Strahlungsweg gehört, der eine Reflexion auf dem Auge des Trägers in der Okularzone, wo sich der linke Abschnitt der Grenze zwischen der Lederhaut und der Iris des Auges verlagert, enthält.

6. Brille nach einem der Ansprüche 1 bis 4, wobei die wenigstens eine Quelle, die Austrittsabschnitte, wenigstens ein Eintrittsabschnitt und der wenigstens eine Detektor, die wenigstens einem der Gläser zugeordnet sind, in der Weise angeordnet sind, dass wenigstens zwei verschiedene Strahlungswege, die durch die verschiedenen Austrittsabschnitte (30, 32, 40, 42) verlaufen, in denselben Eintrittsabschnitt (31), der den Wegen gemeinsam ist, eindringen, und dass die Mittel zum Trennen der Strahlungsanteile als Funktion der Strahlungswege dafür ausgelegt sind, Messungen, die mit dem wenigstens einen Detektor ausgeführt werden, in Übereinstimmung mit den verschiedenen Modulationen, die auf die von den verschiedenen Wegen übertragenen Strahlungen angewendet werden, zu synchronisieren.

7. Brille nach einem der Ansprüche 1 bis 6, wobei jede Strahlungsquelle und jeder Detektor mikrooptoelektronische Bauteile auf der Grundlage von Halbleitermaterial, die in das entsprechende Glas integriert sind, sind, wobei das Glas außerdem transparente Leiterbahnen (40-48) umfasst, die die Anschlüsse jedes Detektors und jeder Quelle mit der Recheneinheit elektrisch verbinden und in einem Umfangsteil des Glases radial verlaufen.

8. Brille nach einem der Ansprüche 1 bis 6, wobei sich jede Strahlungsquelle außerhalb des entsprechenden Glases befindet und mit einem der Austrittsabschnitte (10, 12, 20, 22) über einen ersten Lichtleiter (50, 52, 60, 62) optisch gekoppelt ist und jeder Detektor sich außerhalb des entsprechenden Glases befindet und mit einem der Eintrittsabschnitte (11, 13, 21, 23) durch einen zweiten Lichtleiter (51, 53, 61, 63) optisch gekoppelt ist.

9. Brille nach einem der Ansprüche 1 bis 6, die außerdem für jedes Glas (1, 2) einen Lichtleiter (70) umfasst, der ein Bild eines Teils des Auges (100, 200) außerhalb des Glases erzeugt, wobei die Detektoren in den Zonen des Bildes, wo sich die rechten und linken Abschnitte der Grenze (L) zwischen der Lederhaut (S) und der Iris (1) des Auges verlagern, die bei den Okularbewegungen abgebildet werden, verteilt sind.

10. Brille nach einem der vorhergehenden Ansprüche, die außerdem Mittel zum Verändern einer Charakteristik wenigstens eines der Gläser als Funktion eines Ergebnisses der Konvergenz der Augen des Trägers, die durch die Recheneinheit gekennzeichnet wird, umfasst.

11. Brillenglas (1, 2), das Folgendes umfasst:
- wenigstens eine Strahlungsquelle (10, 12, 20, 22), wobei jede Quelle in das Glas integriert ist und so gewählt ist, dass die Strahlung durch eine Lederhaut (S) und durch eine Iris (I) eines Auges (100, 200) eines Trägers des Glases in Übereinstimmung mit jeweiligen verschiedenen Reflexionsintensitätskoeffizienten für die Lederhaut bzw. für die Iris reflektiert wird;
- wenigstens einen Detektor für die Strahlung (11, 13, 21, 23), wobei jeder Detektor in das Glas integriert ist und dafür ausgelegt ist, eine Intensität eines Strahlungsanteils, der von einer der Quellen erzeugt und durch das Auge in einer Okularzone (ZD1, ZG1, ZD2, ZG2), in der sich ein rechter oder linker Abschnitt einer Grenze (L) zwischen der Lederhaut und der Iris des Auges bei Okularbewegungen verlagert, reflektiert wird, zu messen;
- transparente leitende Bahnen (40-48), die in das Glas integriert sind und Anschlüsse jedes Detektors und jeder Quelle elektrisch verbinden und radial in einem Umfangsteil des Glases verlaufen; und
- wenigstens vier Austritts- oder Eintrittsabschnitte für Strahlung, die in dem Glas angeordnet sind, wobei jeder Austrittsabschnitt (10, 12, 20, 22) dafür ausgelegt ist, einen Strahlungsanteil, der von der Quelle erzeugt wird, zu wenigstens einer der Okularzonen zu lenken, und jeder Eintrittsabschnitt (11, 13, 21, 23) dafür ausgelegt ist, einen Strahlungsanteil, der in einer der Okularzonen reflektiert wird, zu sammeln;
derart, dass wenigstens zwei Strahlungswege gebildet werden, wovon jeder eine Reflexion am Auge des Trägers, das sich hinter dem Glas befindet, in einer der Okularzonen, wo sich die rechten und linken Abschnitte für den einen bzw. den anderen der Wege der Grenze zwischen der Lederhaut und der Iris des Auges verlagern, enthält;
wobei zwei erste Strahlungsaustrittsabschnitte (10, 12) auf derselben ersten Höhe (α₁) im Glas angeordnet sind und zwei zweite Strahlungsaustrittsabschnitte (20, 22) in dem Glas auf derselben zweiten Höhe (α₂), die von der ersten Höhe verschieden ist, angeordnet sind.

12. Brillenglas nach Anspruch 11, das ebenso viele Strahlungsquellen (10, 12, 20, 22) wie Detektoren (11, 13, 21, 23) enthält, wobei jede der Quellen in dem Glas neben einem der Detektoren angeordnet ist, um ein getrenntes Strahlungsaussende/Strahlungsempfangs-Paar zu bilden, wobei jedes Aussende/Empfangs-Paar vor einer der Okularzonen (ZD1, ZG1, ZD2, ZG2) angeordnet ist, wo sich einer der rechten und linken Abschnitte der Grenze (L) zwischen der Lederhaut (S) und der Iris (I) des Auges des Trägers bei Okularbewegungen verlagert.

13. Brillenglas nach Anspruch 11 oder 12, wobei die Quellen in Form von zwei Säulen angeordnet sind, die sich im rechten bzw. im linken Abschnitt des Glases befinden, wobei jede Quelle der Säule (10₁, ..., 10ₙ) , die sich im rechten Abschnitt des Glases (1) befindet, zu einem Strahlungsweg gehört, der eine Reflexion auf dem Auge (100) des Trägers in einer Okularzone, wo sich der rechte Abschnitt der Grenze (L) zwischen der Lederhaut (S) und der Iris (I) des Auges verlagert, enthält, und jede Quelle der Säule (12₁, ..., 12ₙ), die sich in dem linken Teil des Glases befindet, zu einem Strahlungsweg gehört, der eine Reflexion auf dem Auge des Trägers in der Okularzone, wo sich der linke Abschnitt der Grenze zwischen der Lederhaut und der Iris des Auges verlagert, enthält.

14. Brillenglas nach einem der Ansprüche 11 bis 13, wobei jede Strahlungsquelle (10, 12, 20, 22) eine Leuchtdiode ist, die mit einer Wellenlänge im Bereich von 900 nm bis 1200 nm arbeitet.

15. Brillenglas nach einem der Ansprüche 11 bis 14, wobei jeder Detektor (11, 13, 21, 23) eine Photodiode oder ein Phototransistor ist.

16. Brillenglas nach einem der Ansprüche 11 bis 15, das außerdem einen Glasbasisteil (50) und einen Glaseinkapselungsteil (51) umfasst, wobei die Basis- und Einkapselungsteile fest miteinander verbunden sind, wobei jede Strahlungsquelle (10, 12, 20, 22), jeder Detektor (11, 13, 21, 23) und jede Leiterbahn (40-48) des Glases zwischen den Basis- und Einkapselungsteilen angeordnet sind.

17. Brillenglas nach einem der Ansprüche 11 bis 16, das außerdem Mittel zum Verändern einer Charakteristik des Glases umfasst.

## Claims

1. Pair of ophthalmic spectacles, comprising a frame (3) and two lenses (1, 2) held in the frame so as to be situated respectively in front of the eyes (100, 200) of a wearer of the said spectacles, and allowing distinct vision for the wearer through each lens, the pair of spectacles comprising for each lens:
- at least one radiation source selected so that the said radiation is reflected by the sclerotic (S) and by the iris (I) of each eye of the wearer in accordance with different reflection intensity coefficients respectively for the sclerotic and for the iris;
- at least one detector disposed so as to measure respective intensities of parts of the radiation produced by the source, which parts are reflected respectively in ocular zones (ZD1, ZG1, ZD2, ZG2) in each of which a right or left portion of a boundary (L) between the sclerotic and the iris of the eye situated behind the said lens moves during ocular movements; and
- at least four radiation exit or entry sections disposed in the lens, each exit section (10, 12, 20, 22) being disposed so as to direct a part of the radiation produced by the source towards at least one of the said ocular zones, and each entry section (11, 13, 21, 23) being disposed so as to collect a radiation part reflected in one of the said ocular zones;
so as to form at least four radiation paths each comprising a reflection on the eye of the wearer situated behind the said lens, in one of the ocular zones where the right and left portions, respectively for one and the other of the said paths, of the boundary between the sclerotic and the iris of the said eye move;
the pair of spectacles furthermore comprising means for separating the radiation parts transmitted between the said at least one radiation source and the said at least one detector respectively by different radiation paths; and
the pair of spectacles furthermore comprising a calculation unit adapted for characterizing a convergence of the eyes of the wearer towards a common observation point (C), as a function of the intensities measured simultaneously for the two lenses;
for each lens (1, 2), the said at least one source, at least one detector and at least four radiation exit zones (10, 12, 20, 22) or entry zones (11, 13, 21, 23) being disposed so as to form at least two pairs of distinct paths for the radiation, with two first radiation exit sections (10, 12) which are disposed in each lens at one and the same first height (α₁), and two second radiation exit sections (20, 22) which are disposed in each lens at one and the same second height (α₂) different from the said first height,
in such a way that the ocular zones (ZD1, ZG1, ZD2, ZG2) where the right and left portions, respectively for one and the other of the paths of each pair, of the boundary (L) between the sclerotic and the iris of the eye of the wearer situated behind the said lens move, are situated at one and the same height (α₁, α₂) of ocular zones, which height is associated with the said pair, and the two pairs of paths being associated with respective heights of ocular zones that are distinct and common to the two lenses;
and the calculation unit being adapted for characterizing the convergence of the eyes of the wearer as a function of the intensities measured simultaneously for the radiation parts transmitted by the paths associated with one and the same of the heights of ocular zones, which height is selected by the said calculation unit for the two lenses.

2. Pair of spectacles according to Claim 1, in which the calculation unit is adapted for selecting one of the heights (α₁, α₂) of ocular zones (ZD1, ZG1, ZD2, ZG2) to characterize the convergence of the eyes of the wearer, as a function of a value of a signal/noise ratio of at least some of the measured intensities.

3. Pair of spectacles according to Claim 1 or 2, in which the calculation unit is adapted for firstly determining an angular position of an optical axis (D1, D2) of each eye (100, 200) on the basis of the intensities measured simultaneously for the lens (1, 2) situated in front of the said eye, and for radiation parts reflected in some of the ocular zones (ZD1, ZG1, ZD2, ZG2) where the right and left portions of the boundary (L) between the sclerotic (S) and the iris (I) of the said eye move;
and the calculation unit is furthermore adapted for thereafter determining the convergence of the eyes of the wearer on the basis of the respective angular positions of the optical axes of the two eyes determined for one and the same instant.

4. Pair of spectacles according to any one of Claims 1 to 3, comprising as many radiation exit sections (10, 12, 20, 22) as radiation entry sections (11, 13, 21, 23) for each lens, each of the said exit sections being juxtaposed in the lens with one of the said entry sections to form a separate radiation emission-reception pair, each emission-reception pair being disposed in front of one of the ocular zones (ZD1, ZG1, ZD2, ZG2) where one of the right and left portions of the boundary (L) between the sclerotic (S) and the iris (I) of the eye of the wearer situated behind the said lens moves during the ocular movements.

5. Pair of spectacles according to any one of Claims 1 to 4, in which, for each lens (1, 2), the radiation exit sections are disposed in the form of two columns situated respectively in right and left parts of the said lens, each exit section of the column (10₁,..., 10ₙ) situated in the right part of the lens belonging to a radiation path comprising a reflection on the eye (100) of the wearer in the ocular zone where the right portion of the boundary (L) between the sclerotic (S) and the iris (I) of the said eye moves, and each exit section of the column (12₁,..., 12ₙ) situated in the left part of the lens belonging to a radiation path comprising a reflection on the eye of the wearer in the ocular zone where the left portion of the said boundary between the sclerotic and the iris of the eye moves.

6. Pair of spectacles according to any one of Claims 1 to 4, in which the said at least one source, the exit sections, at least one entry section and the said at least one detector which are associated with at least one of the lenses, are disposed so that at least two different radiation paths passing through different exit sections (30, 32, 40, 42) penetrate via one and the same entry section (31) common to the said paths, and the means for separating the radiation parts as a function of the radiation paths are adapted for synchronizing measurements carried out by the said at least one detector in accordance with different modulations applied to the radiations transmitted by the said different paths.

7. Pair of spectacles according to any one of Claims 1 to 6, in which each source of the radiation and each detector are micro-optoelectronic components based on semiconductor material which are integrated into the corresponding lens, and the said lens furthermore comprises transparent conducting tracks (40-48) electrically linking terminals of each detector and of each source to the calculation unit, and which are directed radially in a peripheral part of the lens.

8. Pair of spectacles according to any one of Claims 1 to 6, in which each radiation source is situated outside the corresponding lens, and is coupled optically to one of the exit sections (10, 12, 20, 22) by a first optical guide (50, 52, 60, 62), and each detector is situated outside the corresponding lens, and is coupled optically to one of the entry sections (11, 13, 21, 23) by a second optical guide (51, 53, 61, 63).

9. Pair of spectacles according to any one of Claims 1 to 6, furthermore comprising, for each lens (1, 2), an optical guide (70) which forms an image of a part of the eye (100, 200) outside the said lens, and in which the detectors are distributed in zones of the said image wherein move the right and left portions of the boundary (L) between the sclerotic (S) and the iris (I) of the said eye which are imaged during the ocular movements.

10. Pair of spectacles according to any one of the preceding claims, furthermore comprising means for varying a characteristic of at least one of the lenses, as a function of a result of the convergence, **characterized by** the calculation unit, of the eyes of the wearer.

11. Spectacles lens (1, 2) comprising:
- at least one radiation source (10, 12, 20, 22), each source being integrated into the said lens and selected so that the said radiation is reflected by a sclerotic (S) and by an iris (I) of an eye (100, 200) of a wearer of the lens, in accordance with different reflection intensity coefficients respectively for the sclerotic and for the iris;
- at least one detector of the said radiation (11, 13, 21, 23), each detector being integrated into the said lens and disposed so as to measure an intensity of a part of the radiation produced by one of the sources and reflected by the eye in an ocular zone (ZD1, ZG1, ZD2, ZG2) in which a right or left portion of a boundary (L) between the sclerotic and the iris of the said eye moves during ocular movements;
- transparent conducting tracks (40-48) integrated into the said lens, electrically linking terminals of each detector and of each source, and which are directed radially in a peripheral part of the lens; and
- at least four radiation exit or entry sections disposed in the lens, each exit section (10, 12, 20, 22) being disposed so as to direct a part of the radiation produced by the source towards at least one of the said ocular zones, and each entry section (11, 13, 21, 23) being disposed so as to collect a radiation part reflected in one of the said ocular zones;
so as to form at least two radiation paths each comprising a reflection on the eye of the wearer situated behind the said lens, in one of the ocular zones where the right and left portions, respectively for one and the other of the said paths, of the boundary between the sclerotic and the iris of the said eye move;
with two first radiation exit sections (10, 12) which are disposed at one and the same first height (α₁) in the lens, and two second radiation exit sections (20, 22) which are disposed in the said lens at one and the same second height (α₂) different from the said first height.

12. Spectacles lens according to Claim 11, comprising as many radiation sources (10, 12, 20, 22) as detectors (11, 13, 21, 23), each of the sources being juxtaposed in the lens with one of the detectors to form a separate radiation emission-reception pair, each emission-reception pair being disposed in front of one of the ocular zones (ZD1, ZG1, ZD2, ZG2) where one of the right and left portions of the boundary (L) between the sclerotic (S) and the iris (I) of the eye of the wearer moves during the ocular movements.

13. Spectacles lens according to Claim 11 or 12, in which the sources are disposed in the form of two columns situated respectively in right and left parts of the said lens, each source of the column (10₁,..., 10ₙ) situated in the right part of the lens (1) belonging to a radiation path comprising a reflection on the eye (100) of the wearer in the ocular zone where the right portion of the boundary (L) between the sclerotic (S) and the iris (I) of the said eye moves, and each source of the column (12₁, ..., 12ₙ) situated in the left part of the lens belonging to a radiation path comprising a reflection on the eye of the wearer in the ocular zone where the left portion of the said boundary between the sclerotic and the iris of the eye moves.

14. Spectacles lens according to any one of Claims 11 to 13, in which each source of the radiation (10, 12, 20, 22) is a light-emitting diode operating at a wavelength lying between 900 nm and 1200 nm.

15. Spectacles lens according to any one of Claims 11 to 14, in which each detector (11, 13, 21, 23) is a photodiode or a phototransistor.

16. Spectacles lens according to any one of Claims 11 to 15, furthermore comprising a base part of the lens (50) and a part for encapsulating the lens (51), the said base part and encapsulating part being securely attached, and each source of the radiation (10, 12, 20, 22), each detector (11, 13, 21, 23) and each conducting track (40-48) of the said lens being disposed between the said base part and encapsulating part.

17. Spectacles lens according to any one of Claims 11 to 16, furthermore comprising means for varying a characteristic of the said lens.
